# EUROPEAN PATENT APPLICATION

(11) **EP 3 689 368 A1**
(43) Date of publication of application: **05.08.2020**
(21) Application number: 18860241.1
(22) Date of filing: 24.07.2018
(51) Int. Cl.: A61K 38/48, A61K 33/30

(54) **BOTULINUM TOXIN COMPOSITION HAVING PROLONGED EFFICACY DURATION**

(30) Priority: 29.09.2017 KR 20170126692
(71) Applicant: Korea Prime Pharm Co., Ltd., Gwangju 61473 (KR)
(72) Inventor: KIM, Daeik, Gwangju 61402 (KR); CHO, Yong-Baik, Uiwang-si Gyeonggi-do 16035 (KR); SEO, Inra, Suwon-si Gyeonggi-do 16496 (KR)
(74) Representative: Bringer IP
(86) International application number: PCT/KR2018/008312
(87) International publication number: WO 2019/066227

(57) **Abstract**

The present invention relates to a novel botulinum toxin composition having increased safety and reduced side effects. The composition of the present invention is prepared by adding zinc to a botulinum toxin. The composition, of the present invention, containing a botulinum toxin and zinc, has been confirmed to increase the survival rate of rats and have a greatly prolonged toxin efficacy duration when intramuscularly administered to the rats.

## Description

### TECHNICAL FIELD

The present invention relates to a composition comprising a botulinum toxin. In particular, the present invention relates to a botulinum toxin composition having increased safety/efficacy and extended efficacy duration.

### BACKGROUND ART

Botulinum toxin is produced from *Clostridium botulinum,* an anaerobic, gram-positive bacterium, and is a potent polypeptide neurotoxin. These neurotoxins cause neuroparalytic disorders in humans and animals. *Clostridium botulinum* is found in soil but can be cultured in a sealed food container without being adequately sterilized. Botulinum toxins have a high affinity for cholinergic neurons, and are known to enter neurons and inhibit presynaptic release of acetylcholine. Ingestion of a botulinum toxin results in several disorders, for example, gait disorders, deglutition disorders, and verbal disorder symptoms, and may lead to death due to the paralysis of the respiratory muscle. In particular, botulinum toxin type A is lethal. Based on the molar amount, botulinum toxin type A is lethal, 18 billion times the diphtheria, six billion times sodium cyanide, three thousand times cobrotoxin, and one thousand two million times the chimera (Singh et al. Critical Aspect of Bacterial Protein Proteins Diabetes, Page 63-84 (Chapter 4) of Natural Toxins II, edited by B.R. Sigh et al, Plenum Press, New York (1976).

Botulinum neurotoxin is divided into serotype A, B, C1, D, E, F, and G. Botulinum toxin type A has been approved by the US Food and Drug Administration to be used in the treatment of Essential Blepharospasm, strabismus and hemifacial spasm in patients at least 12 years old. The clinical effect of injected botulinum toxin type A in peripheral muscle usually appears within one week after the injection. The duration of the efficacy by intramuscular injection of botulinum toxin type A is generally about 3 months. In general, the botulinum toxin 1 unit (U) can be defined as the LD₅₀ via injection in the peritoneal cavity in a female Swiss Webster mouse with a weight of 18 - 20 g.

Although all the botulinum toxin serotypes are believed to inhibit release of neurotransmitter acetylcholine at the neuromuscular junction, they function in different neurosecretory proteins. Botulinum toxin type A is a zinc endopeptidase which can specifically hydrolyze peptide bonds of intracellular vesicle-related protein SNAP-25. Botulinum toxin type A is 500 times more potent than botulinum toxin type B in rats. Additionally, botulinum toxin type B is non-toxic in primate at a dose of 480 U/kg, which is about 12 times the primate LD₅₀ of Type A toxin. The botulinum toxin is a component of a toxin complex comprising about 150 kD botulinum toxin protein molecule and a non-toxin protein bound thereto. Pre-prepared and purified botulinum toxin or toxin complexes suitable for use in the preparation of pharmaceutical compositions could be obtained from Metabiologics, Inc., USA, and List Biological Laboratories, Inc. (Campbell, California; the Center for Applied Microbiology and Research, Porton Down Task, U.K.) and the like. The pure botulinum toxin is unstable, and a botulinum toxin complex, such as a toxin type A complex, is very sensitive to denaturation due to surface modification, heat and alkaline conditions.

The botulinum toxin has a wide variety of functions, including esthetic conditions, spasm of the blepharomas, unilateral facial spasm, spasm stiffness, stiffness, muscle tension abnormalities, migraine, low back pain, cervical disease, strabismus, hyperhidrosis and excess saliva secretion, and the like, and active studies of the medical use of botulinum toxins are being carried out. Also, studies on botulinum toxins formulations with increased safety/stability are steadily being performed. Korean Patent Publication No. 10-2008-0050636 discloses a therapeutic composition comprising a botulinum neurotoxin, and Korean Patent Publication No. 10-2007-0110402 discloses a composition for topical application and transdermal delivery of a botulinum toxin. Also, Korean Patent Application No. 10-2016-008798 describes extending the duration of toxin potency by using a hyaluronic acid filler together with a botulinum toxin. Korean Patent Laid-Open Publication No. 10-2015-0057327 describes stabilizing botulinum toxins for a long period of time by the encapsulation of them in a phospholipid micelle formed by the combination of surfactants, solvents, stabilizers, and at least one absorption enhancer selected from the group consisting of collagen, elastin, short chain alcohols, long chain alcohols, or polyalcohols, amines, and amides.

As such, the botulinum toxin exhibits fatal toxicity even in trace amounts and can cause side effects. The present inventors have studied to increase the safety of a botulinum toxin and to prolong the duration of efficacy while reducing side effects. As a result, it was surprisingly found that when zinc was added to the botulinum toxin composition, the duration of the botulinum toxin efficacy was extended and the side effects were greatly reduced.

### DETAILED DESCRIPTION OF THE INVENTION

### TECHNICAL PROBLEM

The purpose of the present invention is to provide a novel botulinum toxin composition which has increased safety/efficacy and has an extended efficacy duration.

### TECHNICAL SOLUTION

To solve the technical problems above, the present invention provides a botulinum toxin composition comprising botulinum toxin and zinc.

In an embodiment of the present invention, the duration of botulinum toxin efficacy was observed with varying amounts of zinc added. A sample of a botulinum toxin composition containing progressively increased amounts of zinc was prepared and administered to rats, and then the duration of toxin efficacy was confirmed. As a result, it was confirmed that the duration of the botulinum toxin potency increased with the amount of zinc added. The amount of zinc added to extend the duration of the botulinum toxin efficacy is preferably 0.001 mM/100U to 3.0 mM/100U for the botulinum toxin 100 unit (U), more preferably 0.001 mM/100U to 0.33 mM/100U for 100 U of botulinum toxin. Thus, the amount of zinc added to extend the duration of the botulinum toxin efficacy per botulinum toxin unit (U) is preferably from 0.01 µM/U to 30 µM/U, and more preferably 0.01 µM/U to 3.3 µM/U.

As used herein, the term "botulinum toxin" means a botulinum toxin protein molecule of about 150 kD including all serotypes and variants or fusion proteins thereof produced by bacteria or recombination. Botulinum toxin serotypes are available, for example, from Sigma-Aldrich (St Louis, MO) and Metabiologics, Inc. (Madison, Wisconsin) and the like. The different serotypes of botulinum toxin differ in the subject animal, efficacy and duration.

The botulinum toxin used in the compositions of the present invention comprises a botulinum toxin derivative. A botulinum toxin derivative may be a natural botulinum toxin having a botulinum toxin activity or a derivative comprising one or more chemical or functional modifications on a portion or some chain of a recombinant botulinum toxin. For example, a botulinum toxin derivative can be a modified toxin having one or more amino acids that are deleted, modified, or substituted. In the present invention, the botulinum toxin can be a recombinant peptide, a fusion protein, or a hybrid neurotoxin made from, for example, subunits or domains of different toxin serotypes. A botulinum toxin can also be a part of an entire molecule having toxin activity, or can be used as a part of a combination or a conjugation of the molecule, e.g., a fusion protein.

The composition of the present invention is preferably present in the form of a product to be applied to the skin, epithelium, intradermal (ID), subcutaneous (SC), intramuscular (IM), and intravascular (IM) of a human or other mammals in need of administration of a botulinum toxin. The term "requiring" is intended to include pharmaceutical or health needs, such as, the need to treat symptoms including facial muscle spasm, and to include cosmetic and subjective needs, such as, the need to modify or improve the appearance of facial tissue. In general, the composition can be prepared by mixing a botulinum toxin and one or more pharmaceutically acceptable carriers or excipients. The compositions of the present invention may include solutions, emulsions (including microemulsions), suspensions, creams, lotions, gels, powders, or other solid or liquid compositions used for application to skin or other tissues in which the compositions of the present invention may be applied. Such compositions, in addition to botulinum toxin, may comprise other generally used ingredients, such as antimicrobial agents, moisturizers and hydrating agents, permeation agents, preservatives, emulsifiers, natural or synthetic oils, solvents, surfactants, detergents, gelling agents, emollients, antioxidants, fragrances, fillers, thickeners, waxes, odor absorbers, dyes, colorants, powders, viscosity modifiers, and water, and optionally, anesthetics, antiitch actives, plant extracts, conditioning agents, darkening agents or lightening agents, a glitter, a humectant, a mica, a mineral, a polyphenol, a silicone or a derivative thereof, a sunblock, a vitamin, and a phytomedicine. Th botulinum toxin can be administered to the subcutaneous muscle or glandular structure in the skin in an effective amount to induce paralysis or relaxation, reduce contraction, prevent or reduce spasm, reduce glandular output, or other effects. The composition of the present invention is applied such that an effective amount of a botulinum toxin is administered and its efficacy is maintained for a long time.

As used herein, the term "effective amount" means the amount of botulinum toxin that is sufficient to produce the desired muscle paralysis or other biological or cosmetic effects, while the amount is safe enough to prevent serious side effects. The desired effect includes, for example, adjusting the appearance of the face, for example, by reducing fine lines and/or wrinkles or increasing the corner of the mouth, or relaxing some muscles for the purpose of general relief of muscle tension. The compositions of the present invention may comprise a suitable dosage of botulinum toxin for a single dosage application, or more concentrated for dilution at the site of administration or for use in multiple applications. The site of administration of the botulinum toxin may be legs, shoulders, back (including waist), armpit, palm, foot, neck, groin, the back of the hand, the top of the foot, elbow, upper arm, knee, upper leg, hip, torso, pelvis, or other portions of the body where administration of a botulinum toxin is desired. Administration of a botulinum toxin can also be carried out for the treatment of a neuropathic pain, prevention or alleviation of migraine or other headache, prevention or alleviation of acne, prevention or alleviation of symptoms associated with dystonia or muscle contraction (subjective or clinical), prevention or alleviation of symptoms associated with subjective or clinical hyperhidrosis, alleviation of hypersecretion or perspiration, reduction or enhancement of an immune response, or treatment of any other conditions for which administration of a botulinum toxin by injection is suggested or performed, but not limited thereto.

### EFFECT OF THE INVENTION

The present invention provides a botulinum toxin composition having increased safety, prolonged efficacy, and significantly reduced side effects. The botulinum toxin composition of the present invention can be administered to a subject of a patient in need of administration of a botulinum toxin with high safety, thereby widening the application of the botulinum toxin.

### BRIEF DESCRIPTION OF DRAWINGS

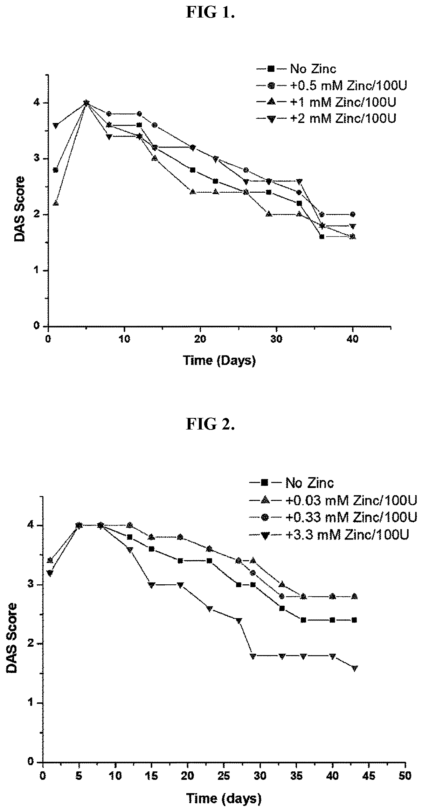

FIGS. 1-4 show the administration of a botulinum toxin composition containing different concentrations of zinc to rats and confirming the duration of botulinum toxin efficacy.

### BEST MODE FOR INVENTION

The present invention will now be described in more detail based on the examples below. The following examples are intended to better understand the invention and are not intended to limit the scope of the invention by these examples.

### Example: Confirmation of enhancement of botulinum toxin efficacy by zinc addition

To analyze how the zinc ions affect the efficacy of the botulinum toxin, the muscle paralysis was measured following administration of a botulinum toxin (Ron S. Broide et al., Toxicon, 2013, 71; 18-24). First, botulinum toxin compositions with various concentrations of zinc (ZnCh) was prepared. The botulinum toxin compositions were prepared by diluting a botulinum toxin at a concentration of 2.7 × 10⁵ LD₅₀ U/µg (1 µg/ml) purchased from Metabiologic Inc. in PBS (phosphate buffered saline solution). Three groups of the botulinum toxin composition samples were prepared by adding zinc (available from Sigma) to the prepared botulinum toxin compositions. The amount of zinc used in each group is as follows: first group - 0.33 mM/100U, 3.33 mM/100U or 33.3 mM/100U; second group - 0.03 mM/100U, 0.33 mM/100U or 3.3 mM/100U; third group - 0.5 mM/100U, 1 mM/100U or 2 mM/100U; and fourth group - 0.001 mM/100U, 0.0033 mM/100U, 0.01 mM/100U, 0.03 mM/100U or 0.33 mM/100U. For all of the above four groups, a toxin diluted with PBS was used as a control. For animal experiments, SD (Sprague-Dawley) rats (Orientbio, 6 weeks, female, 160 - 180 g) were divided into four groups (5 mice/group). The sample prepared above was administered by 100 µl into the lower limb muscle of each rat in respective groups at a concentration of 30 U/kg. The sample was administered once, and after the administration the Digit Abduction Score (DAS) was observed for approximately 60 days based on the following Table 1. The results are shown in FIGS. 1, 2, 3 and 4, respectively.

**[Table 1]**

| DAS score | Symptoms |
|---|---|
| 0 | Normal |
| 1 | big toe abduction occurs |
| 2 | big toe and index toe abductions occur |
| 3 | toes, except little toe, abduction occur |
| 4 | 5 toes have abduction, and all toes are gathered |

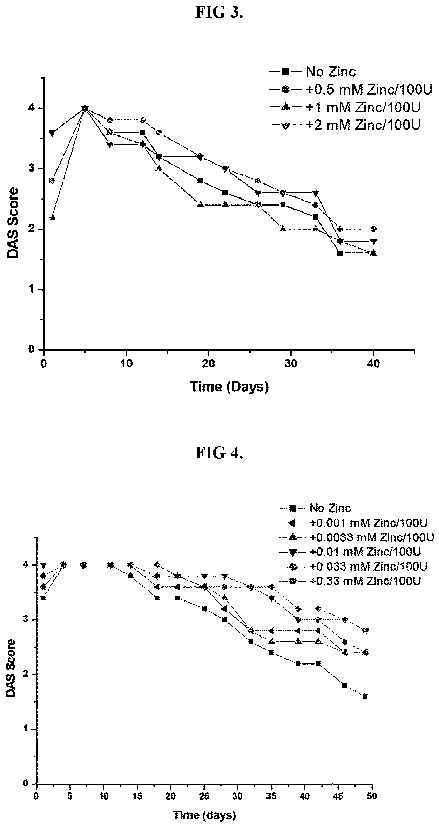

In FIG. 1, a step-wise increased amount (0.33 mM/100U, 3.33 mM/100U, or 33.3 mM/100U) of zinc was added to prepare a sample of a botulinum toxin composition, and the duration of botulinum toxin efficacy was analyzed by the method above (first group). As a result, the efficacy of the botulinum toxin was increased at 0.33 mM/100U, but at other concentrations the zinc addition was confirmed to be independent of the efficacy of the toxin. In FIG. 2, a step-wise increased amount (0.03 mM/100U, 0.33 mM/100U, or 3.3 mM/100U) of zinc was added to prepare a sample of a botulinum toxin composition and then the duration of botulinum toxin efficacy was analyzed by the method above (second group). As a result, zinc concentration of 0.03 mM/100U and 0.33 mM/100U increased the efficacy of the botulinum toxin, but at 3.3 mM/100U, the zinc addition was independent of the increase in toxin efficacy. In addition, in FIG. 3, a step-wise increased amount (0.5 mM/100U, 1 mM/100U, or 2 mM/100U) of zinc was added to prepare a sample of the botulinum toxin composition, and then the duration of the botulinum toxin efficacy was analyzed by the said method (third group). As a result, the zinc concentration was found to be unrelated to the efficacy enhancement of the toxin at 0.5 mM/100U, 1 mM/100U, or 2 mM/100U.

In addition, in FIG. 4, the botulinum toxin composition samples were prepared by adding zinc (0.001 mM/100U, 0.0033 mM/100U, 0.01 mM/100U, 0.03 mM/100U, or 0.33 mM/100U), followed by the analysis of the duration of botulinum toxin efficacy (fourth group). As a result, it was confirmed that the efficacy of the botulinum toxin was increased at a zinc concentration of 0.001 mM/100U or more and 0.33 mM/100U or less.

### Industrial Applicability

According to this invention, botulinum toxin composition, which significantly increases the duration of toxin efficacy by zinc addition, is provided. The botulinum toxin composition of the present invention may be widely used to prepare a composition of various functions for a subject in need of administering a botulinum toxin for cosmetic or medical purposes, or for the prevention or treatment of a disease.

## Claims

1. A botulinum toxin composition comprising botulinum toxin and zinc, **characterized by** having prolonged duration of botulinum toxin efficacy *in vivo.*

2. The botulinum toxin composition of claim 1, wherein the amount of zinc is 0.01 µM/U to 30 µM/U for botulinum toxin unit (U).

3. The botulinum toxin composition of claim 2, wherein the amount of zinc is 0.01 µM/U to 5 µM/U for botulinum toxin unit (U).

4. The botulinum toxin composition according to any one of claims 1 to 3, wherein the botulinum toxin is selected from the group consisting of botulinum toxin types A, B, C1, D, E, F and G.

5. The botulinum toxin composition of claim 4, wherein the botulinum toxin is a botulinum toxin type A.

6. A method of preparing a botulinum toxin composition having prolonged duration of botulinum toxin efficacy *in vivo,* comprising applying zinc to a composition comprising a botulinum toxin, wherein the zinc is applied in an amount of 0.01 µM/U to 30 µM/U for a botulinum toxin unit (U).

7. The method of claim 6, wherein the zinc is applied in an amount of 0.01 µM/U to 5 µM/U.

8. The method of claim 6 or 7, wherein the botulinum toxin is selected from the group consisting of botulinum toxin types A, B, C1, D, E, F and G.

9. The method of claim 8, wherein the botulinum toxin is botulinum toxin type A.
